# EUROPÄISCHE PATENTSCHRIFT

(11) **EP 1 093 397 B1**
(45) Veröffentlichungstag und Bekanntmachung des Hinweises auf die Patenterteilung: **12.11.2003**
(21) Anmeldenummer: 99939776.3
(22) Anmeldetag: 29.05.1999
(51) Int. Cl.: B01F 17/00, C08G 63/664, C07C 69/708

(54) **EMULGATOREN**
EMULSIFIERS
EMULSIFIANTS

(30) Priorität: 08.06.1998 DE 19825462
(43) Veröffentlichungstag der Anmeldung: 25.04.2001
(73) Patentinhaber: Cognis Deutschland GmbH & Co. KG, 40589 Düsseldorf (DE)
(72) Erfinder: ANSMANN, Achim, D-40699 Erkrath (DE); BRÜNING, Stefan, D-40597 Düsseldorf (DE); KAWA, Rolf, D-40789 Monheim (DE); STRAUSS, Gabriele, D-40589 Düsseldorf (DE)
(86) Internationale Anmeldenummer: EP9903740
(87) Internationale Veröffentlichungsnummer: WO99064142

(56) Entgegenhaltungen:
- EP-A- 0 868 902
- DE-A- 19 533 539
- US-A- 5 670 478

## Beschreibung

### Gebiet der Erfindung

Die Erfindung befindet sich auf dem Gebiet der kosmetischen Emulsionen und betrifft Emulgatoren, die Emulsionen eine erhöhte Viskosität und verbesserte Stabilität verleihen.

### Stand der Technik

Polyglycerinpoly-12-hydroxystearate stellen bekannte Emulgatoren für die Herstellung von W/O- bzw. O/W-Emulsionen dar. Die Stoffe werden beispielsweise in den deutschen Offenlegungsschriften **DE-A1 4420516** und **DE-A1 19533539** (Henkel) beschrieben. Zur Verbesserung der Emulsionseigenschaften, insbesondere der Viskosität und der Lagerbeständigkeit, werden Emulgatoren gewöhnlich zusammen mit Metallseifen vom Typ des Magnesium-, Aluminium- oder Zinkstearats eingesetzt. Es hat sich jedoch gezeigt, daß Mischungen von Polyolpoly-12-hydroxystearat und beispielsweise Zinkstearat zu Emulsionen führt, deren Viskosität für eine Reihe von Anwendungen nicht hoch genug ist und die sich bei Lagerung im Bereich von 40 bis 45°C nach einiger Zeit entmischen. Die Aufgabe der vorliegenden Erfindung hat daher darin bestanden, Emulgatoren zur Verfügung zu stellen, die frei von den geschilderten Nachteilen sind.

### Beschreibung der Erfindung

Gegenstand der Erfindung sind Emulgatoren, enthaltend
(a) Polyolpoly-12-hydroxystearate und
(b) Metallseifen von Hydroxycarbonsäuren.

Überraschenderweise wurde gefunden, daß Mischungen von Polyolpoly-12-hydroxystearaten mit Metallseifen von Hydroxycarbonsäuren, insbesondere Mischungen von Polyglycerinpoly-12-hydroxystearaten mit Zinkricinoleat, Emulsionen nicht nur eine höhere Viskosität verleihen, sondern sie auch über den gesamten Temperaturbereich von -5 bis 45°C dauerhaft stabilisieren.

### Polyolpoly-12-hydroxystearate

Bei den Polyolpoly-12-hydroxystearaten handelt es sich um bekannte Stoffe, die beispielsweise unter der Marke Dehymuls® PGPH vertrieben werden. Die Polyolkomponente der Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10, wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Wie schon erwähnt, kommen unter den erfindungsgemäß einzusetzenden Emulgatoren Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammem angegeben sind die bevorzugten Bereiche):

| | |
|---|---|
| Glycerin | 5 bis 35 (15 bis 30) Gew.-% |
| Diglycerine | 15 bis 40 (20 bis 32) Gew.-% |
| Triglycerine | 10 bis 35 (15 bis 25) Gew.-% |
| Tetraglycerine | 5 bis 20 ( 8 bis 15) Gew.-% |
| Pentaglycerine | 2 bis 10 (3 bis 8) Gew.-% |
| Oligoglycerine | ad 100 Gew.-% |

### Metallseifen

Bei den Metallseifen handelt es sich vorzugsweise um die Magnesium-, Aluminium- und/oder Zinksalze der Ricinol- oder 12-Hydroxystearinsäure. Die Salze können alleine oder in beliebigen Mischungen eingesetzt werden. Das Gewichtsverhältnis der beiden Komponenten (a) und (b) kann dabei bezogen auf den Feststoffanteil im Bereich von 20 : 80 bis 80 : 20, vorzugsweise 40 : 60 bis 60 : 40 liegen.

### Alkyl- und/oder Alkenyloligoglykoside

Polyolpoly-12-hydroxystearate stellen primär W/O-Emulgatoren dar. Durch Abmischung mit nichtionischen Tensiden vom Typ der Alkyloligoglucoside läßt sich das Emulgierverhalten jedoch nachhaltig beeinflussen, so daß O/W-Emulgatoren resultieren. Mischungen von Polyglycerinpoly-12-hydroxystearat und Alkylglucosiden sind unter der Marke Eumulgin® VL 75 im Handel erhältlich. In einer besonderen Ausführungsform der Erfindung, die vorzugsweise auf die Herstellung von O/W-Emulsionen gerichtet ist, werden daher als dritte Komponente (c) Alkyl- und Alkenyloligoglykoside mitverwendet. Diese stellen bekannte nichtionische Tenside dar, die der Formel **(l)** folgen,

**R**^{**1**}**O[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in **Starch/Stärke 45, 281 (1993),** B.Salka in **Cosm.Toil. 108, 89 (1993)** sowie J.Kahre et al. in **SÖFW-Journal Heft 8, 598 (1995)** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside.** Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3. Die Einsatzmenge der Alkyl- und/oder Alkenyloligoglykoside beträgt üblicherweise 0,1 bis 10 und vorzugsweise 1 bis 5 Gew.-% - bezogen auf die Zubereitungen. Die Komponenten (a) und (c) können dabei im Gewichtsverhältnis 90 : 10 bis 10 : 90, vorzugsweise 75 : 25 bis 25 : 75 und insbesondere 60: 40 bis 40 : 60 eingesetzt werden.

Demzufolge können die Emulgatoren, je nachdem, ob sie für die Herstellung von W/O- oder O/W-Emulsionen eingesetzt werden sollen,
(a) 20 bis 80, vorzugsweise 40 bis 60 Gew.-% Polyolpoly-12-hydroxystearate,
(b) 80 bis 20, vorzugsweise 40 bis 60 Gew.-% Metallseifen von Hydroxycarbonsäuren und
(c) 0 bis 30, vorzugsweise 10 bis 20 Gew.-% Alkyl- und/oder Alkenyloligoglykoside
enthalten, mit der Maßgabe, daß sich die Mengenangaben auf den Feststoffanteil beziehen und sich zu 100 Gew.-% ergänzen. Die Einsatzmenge der Emulgatoren, die ihrerseits einen nichtwäßrigen Anteil von 30 bis 100 und vorzugsweise 50 bis 70 Gew.-% aufweisen können, kann - bezogen auf die Endformulierungen - 1 bis 20, vorzugsweise 2 bis 10 und insbesondere 5 bis 8 Gew.-% betragen.

### Gewerbliche Anwendbarkeit

Unter Einsatz der erfindungsgemäßen Emulgatoren lassen sich eine Vielzahl von kosmetischen Zubereitungen herstellen, wie beispielsweise Haarshampoos, Haarlotionen, Schaumbäder, Cremes, Lotionen oder Salben. Die Formulierungen können neben den Emulgatoren milde Tenside, Ölkörper, Co-Emulgatoren, Überfettungsmittel, Perlglanzwachse, Konsistenzgeber, Verdickungsmittel, Polymere, Siliconverbindungen, biogene Wirkstoffe, Antischuppenmittel, Filmbildner, Konservierungsmittel, Hydrotrope, Solubilisatoren, UV-Lichtschutzfaktoren, Antioxidantien, Insektenrepellentien, Selbstbräuner, Parfümöle, Farbstoffe und dergleichen enthalten.
Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkohoicarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

Als **Co-Emulgatoren** kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Anlagerungsprodukte von 15 bis 60 Mol Ethylenoxid an Ricinusöl und/oder gehärtetes Ricinusöl;
(5) Anlagerungsprodukte von 2 bis 15 Mol Ethylenoxid an Ricinusöl und/oder gehärtetes Ricinusöl;
(6) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(7) Mono-, Di- und Trialkylphosphate sowie Mono-, Di- und/oder Tri-PEG-alkylphosphate und deren Salze;
(8) Wollwachsalkohole;
(9) Polysiloxan-Polyalkyl-Polyether-Copolymere bzw. entsprechende Derivate;
(10) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(11) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N.Ndimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge. und/oder. Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethyl-cellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs. Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. **Quellmittel** können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-13,5-triazin und Octyl Triazon, wie in der **EP-A1 0818450** beschrieben;
- Propan-1,3-dione, wie z.B. 1-(4-tertButylphenyl)-3-(4'methoxyphenyl)propan-1,3-dion;
- Ketotricydo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen. vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktions-kette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester ) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin. A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiver-öl, Olibanöl, Galbanumöl, Labolanumöl und Lavandinöl. Vorzugsweise werden Bergamotteöl, Dihydro-myrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damas-cone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0.001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Die Viskosität und Lagerstabilität verschiedener Emulsionen wurde im Temperaturbereich von -5 bis +45°C geprüft. Die Viskosität wurde nach der Brookfield-Methode (RVT-Viskosimeter, 10 Upm, Spindel 1) bestimmt, die Stabilität auf einer subjektiven Skala beurteilt. Dabei bedeutet (+++) = stabil und klar, (++) = leichte Trübung und (+) = starke Trübung/beginnende Entmischung. Die Beispiele 1 bis 3 sind erfindungsgemäß, die Beispiele V1 bis V3 dienen zum Vergleich. Die Ergebnisse sind in Tabelle 1 zusammengefaßt.

**Tabelle 1**

| **Viskosität und Stabilität von Emulsionen** | | | | | | |
|---|---|---|---|---|---|---|
| **Zusammensetzung/Performance** | **1** | **2** | **3** | **V1** | **V2** | **V3** |
| Polyglyceryl-2 Dipolyhydroxystearate | 3,0 | 3,0 | 2.0 | 3,0 | 3,0 | 2,0 |
| Coco Glucosides | - | - | 1,0 | - | - | 1.0 |
| Polyglyceryl-3 Diisostearate | 1,0 | 1,0 | 1,0 | 1,0 | 1,0 | 1,0 |
| Zinc Ricinoleate | 1,0 | - | 1,0 | - | - | - |
| Zinc 12-Hydroxystearate | - | 1,0 | - | - | - | - |
| Zinc Stearate | - | - | - | 1,0 | - | 1,0 |
| Magnesium Stearate | - | - | - | - | 1,0 | - |
| Hexyl Laurate | 6,0 | 6,0 | 6,0 | 6,0 | 6,0 | 6,0 |
| Dicaprylyl ether | 6,0 | 6,0 | 6.0 | 6,0 | 6,0 | 6,0 |
| Cetearyl Isononanoate | 7.0 | 7,0 | 7.0 | 7,0 | 7,0 | 7,0 |
| Octyldodecanol | 3,0 | 3,0 | 3,0 | 3,0 | 3.0 | 3,0 |
| Glycerol 86 %ig | 5,0 | 5,0 | 5,0 | 5,0 | 5,0 | 5.0 |
| Magnesium Sulfate | 1,0 | 1,0 | 1,0 | 1,0 | 1,0 | 1,0 |
| Wasser, Konservierungsmittel | ad 100 | | | | | |

| ***Viskosität* [mPas]** | | | | | | |
|---|---|---|---|---|---|---|
| *- nach 1 d* | 5.600 | 5.600 | 5.500 | 4.400 | 4.200 | 4.500 |
| *- nach 1 w* | 6.000 | 5.800 | 5.6.00 | 4.400 | 4.300 | 4.500 |

| ***Stabilität nach 1 w*** | | | | | | |
|---|---|---|---|---|---|---|
| *- bei 20°C* | +++ | +++ | +++ | ++ | + | ++ |
| *- bei -5°C* | +++ | +++ | +++ | +++ | ++ | ++ |
| *- bei 40°C* | +++ | +++ | +++ | + | ++ | ++ |
| *- bei 45°C* | +++ | +++ | +++ | + | ++ | ++ |

## Patentansprüche

1. Emulgatoren, enthaltend
(a) Polyolpoly-12-hydroxystearate und
(b) Metallseifen von Hydroxycarbonsäuren.

2. Emulgatoren nach Anspruch 1, **dadurch gekennzeichnet, daß** sie als Komponente (a) Polyglycerinpoly-12-hydroxystearate enthalten.

3. Emulgatoren nach den Ansprüchen 1 oder 2, **dadurch gekennzeichnet, daß** sie als Komponente
(a) Polyglycerinpoly-12-hydroxystearate enthalten, deren Polyglycerinkomponente einen Eigenkondensationsgrad im Bereich von 2 bis 10 und die nachstehende Zusammensetzung aufweist:
| | |
|---|---|
| Glycerin | 5 bis 30 Gew.-% |
| Diglycerine | 15 bis 40 Gew.-% |
| Triglycerine | 10 bis 30 Gew.-% |
| Tetraglycerine | 5 bis 20 Gew.-% |
| Pentaglycerine | 2 bis 10 Gew.-% |
| Oligoglycerine | ad 100 Gew.-% |

4. Emulgatoren nach mindestens einem der Ansprüche 1 bis 3, **dadurch gekennzeichnet, daß** sie als Komponente (b) Magnesium-, Aluminium- oder Zinksalze der Ricinol- und/oder 12-Hydroxystearinsäure enthalten.

5. Emulgatoren nach mindestens einem der Ansprüche 1 bis 4, **dadurch gekennzeichnet, daß** sie als Komponente (c) weiterhin Alkyl- und/oder Alkenyloligoglykoside enthalten.

6. Emulgatoren nach Anspruch 5, **dadurch gekennzeichnet, daß** sie Alkyl- undloder Alkenyloligoglykoside der Formel **(l)** enthalten,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

7. Emulgatoren nach mindestens einem der Ansprüche 1 bis 6, **dadurch gekennzeichnet, daß** sie
(a) 20 bis 80 Gew.-% Polyolpoly-12-hydroxystearate,
(b) 80 bis 20 Gew.-% Metallseifen von Hydroxycarbonsäuren und
(c) 0 bis 30 Gew.-% Alkyl- und/oder Alkenyloligoglykoside
enthalten, mit der Maßgabe, daß sich die Mengenangaben auf den Feststoffanteil beziehen und sich zu 100 Gew.-% ergänzen

## Claims

1. Emulsifiers containing
(a) polyol poly-12-hydroxystearates and
(b) metal soaps of hydroxycarboxylic acids.

2. Emulsifiers as claimed in claim 1, **characterized in that** they contain polyglycerol poly-12-hydroxystearates as component (a).

3. Emulsifiers as claimed in claim 1 or 2, **characterized in that** they contain as component (a) polyglycerol poly-12-hydroxystearates of which the polyglycerol component has a degree of self-condensation of 2 to 10 and the following composition:
| | |
|---|---|
| glycerol | 5 to 30 % by weight |
| diglycerols | 15 to 40 % by weight |
| triglycerols | 10 to 30 % by weight |
| tetraglycerols | 5 to 20 % by weight |
| pentaglycerols | 2 to 10 % by weight |
| oligoglycerols | to 100 % by weight |

4. Emulsifiers as claimed in at least one of claims 1 to 3, **characterized in that** they contain magnesium, aluminium or zinc salts of ricinoleic acid and/or 12-hydroxystearic acid as component (b).

5. Emulsifiers as claimed in at least one of claims 1 to 4, **characterized in that** they contain alkyl and/or alkenyl oligoglycosides as an additional component (c)

6. Emulsifiers as claimed in claim 5, **characterized in that** they contain alkyl and/or alkenyl oligoglycosides corresponding to formula **(I)**:
**R**^{**1**}**O-[G]**_{**p**} **(I)**
where R¹ is an alkyl and/or alkenyl group containing 4 to 22 carbon atoms, G is a sugar unit containing 5 or 6 carbon atoms and p is a number of 1 to 10.

7. Emulsifiers as claimed in at least one of claims 1 to 6, **characterized in that** they contain
(a) 20 to 80% by weight of polyol poly-12-hydroxystearates,
(b) 80 to 20% by weight of metal soaps of hydroxycarboxylic acids and
(c) 0 to 30% by weight of alkyl and/or alkenyl oligoglycosides,
with the proviso that the quantities mentioned are based on the solids content and add up to 100% by weight.

## Revendications

1. Agents émulsionnants contenant
a) Des poly-12-hydroxystéarates de polyol et
b) des savons métalliques d'acides hydroxycarboxyliques.

2. Agents émulsionnants selon la revendication 1,
**caractérisés en ce qu'**
ils contiennent comme composant a) un poly-12-hydroxystéarate de polyglycérol.

3. Agents émulsionnants selon la revendication 1 ou la revendication 2,
**caractérisés en ce que**
comme composants a) ils contiennent des poly-12-hydroxystéarates de polyglycérol dont le composant polyglycérol possède un degré d'auto-condensation dans la zone de 2 à 10, et possédant la composition suivante :
- Glycérol : de 5 à 30 % en poids
- Diglycérol : de 15 à 40 % en poids
- Triglycérol : de 10 à 30 % en poids
- Tétraglycérol : de 5 à 20 % en poids
- Pentaglycérol : de 2 à 10 % en poids
- Oligoglycérols : qsp 100 % en poids.

4. Agents émulsionnants selon au moins une des revendications 1 à 3,
**caractérisés en ce qu'**
ils renferment comme composants b) des sels de magnésium, d'aluminium ou de zinc de l'acide ricinoléique et/ou 12-hydroxystéarique.

5. Agents émulsionnants selon au moins une des revendications 1 à 4,
**caractérisés en ce qu'**
ils renferment comme composants c) en outre des alkyl- et/ou alkényloligoglycosides

6. Agents émulsionnants selon la revendication 5,
**caractérisés en ce qu'**
ils renferment des alkyl et/ou alkényloligoglycosides de formule I :
R¹O-[G]_{P} (I)
dans laquelle R¹ représente un reste alkyle et/ou alkényle ayant de 4 à 22 atomes de carbone, G représente un reste de sucre ayant 5 ou 6 atomes de carbone et p représente des nombres allant de 1 à 10.

7. Agents émulsionnants selon au moins une des revendications 1 à 6,
**caractérisés en ce qu'**
ils renferment
a) de 20 à 80 % en poids de poly-12- hydroxystéarate de polyol,
b) de 80 à 20 % en poids de savons métalliques d'acide hydroxycarboxylique, et
c) de 0 à 30 % en poids d'alkyl- et/ou alkényloligoglycosides,
avec la précision que les données en quantités se rapportent à la proportion de matière solide, et se complètent à 100 % en poids.
